# EUROPEAN PATENT APPLICATION

(11) **EP 3 943 145 A1**
(43) Date of publication of application: **26.01.2022**
(21) Application number: 19920078.3
(22) Date of filing: 30.09.2019
(51) Int. Cl.: A61M 35/00, A61M 1/00

(54) **SURGICAL AID DEVICE FOR SUTURELESS CLOSURE OF SKIN WOUND IN SUPERFICIAL FASCIA OF SKIN**

(30) Priority: 19.03.2019 CN 201910208814
(71) Applicant: Jingrun (Shanghai) Medical Instruments Co., Ltd., Pudong New District, Shanghai 201306 (CN)
(72) Inventor: CHEN, Wei, Shanghai 201306 (CN)
(74) Representative: Herzog, Markus
(86) International application number: PCT/CN2019/109564
(87) International publication number: WO 2020/186728

(57) **Abstract**

A surgical aid device for the sutureless closure of a skin wound in the superficial fascia of a skin, comprising: a subcutaneous negative pressure drainage device (1) comprising a catheter (10) partially inserted into a subcutaneous wound cavity (4) by a preset depth to generate a negative pressure to make the subcutaneous wound cavity (4) close during a healing process; a wound closure device (2) provided around a skin wound to press the skin edge to keep the skin wound in a closed state during the healing process; and an epidermic negative pressure device (3) provided on the periphery of the skin wound to generate a negative pressure to keep tissue positions of the skin wound and peripheral area thereof stable. The surgical aid device can implement the sutureless closure of full-thickness tissue above the deep fascia of the skin, avoids horizontal scars on the skin surface caused by suture compression/cutting, and leaves no suture knots in the superficial fascia, thereby eliminating an important element which causes colonization of bacteria and a main cause of recurrence of wound infection.

## Description

### TECHNICAL FIELD

The present application relates to the field of medical apparatus and instruments, particularly to surgical auxiliary equipment for sutureless closed skin incisions in the superficial fascia of the skin.

### BACKGROUD ART

As we all know, humans have gradually lost an ability to regenerate a body part in the process of evolution. Scar healing is the main way to deal with tissue damage caused by trauma. Therefore, there is a close relationship between surgical suturing and tissue healing. To a certain extent, the quality of suturing can determine the quality of tissue healing. It is common to use a curved needle carrying sutures pass through the transecting tissues in surgical suturing, and then tighten and fix the sutures by knotting, so that the transecting tissues fit closely, thereby creating favorable conditions for healing. The ideal surgical suturing should meet the requirements of moderate tension, good incision edge alignment, no dead space, no permanent or only a few sutured marks. According to the structure and physiological characteristics of different tissues, the requirements for surgical suture are different, the selection of suturing materials and manners greatly affects the surgical effect. When the tissue is healed and its functional strength is restored, the suturing material loses its functional significance. At this moment, the sutures on the body surface should be removed. Large numbers of clinical medicine prove that it is difficult to remove the sutures left in the deep tissues caused by suturing after the incision is healed. The result is that the sutures will be wrapped by scar tissues, or being degraded and absorbed within a certain period of time. These knots left in the deep tissues sometimes affect the human body. In addition, the significant scars caused by traditional surgical suturing also have a great impact on aesthetics. Currently, there are the following ways to avoid the suture scars on the surface of the skin: 1, intradermal suture method: after completing the suturing of superficial fascia, a surgeon uses sutures to make continuous intradermal suturing in the superficial layer of dermis, and tighten the sutures to align the edge of skin; 2, tissue glue method: also after the superficial fascia suturing is completed, the edge of the skin is adhered with methyl acrylate tissue glue; 3, skin tension reducer (commonly known as a skin zipper): after the superficial fascia suturing is completed, a tension reducing tape with a locking structure is used to pull the skin tightly and tighten the edge of the skin. However, as to the current types of solutions, a large number of suturing in the superficial fascia layer of the skin is still needed, which cannot avoid a series of safety problems caused by residual sutures. Safe and aesthetically pleasing closed skin incisions have become a technical problem that those skilled in the art are expected to solve.

### SUMMARY OF THE INVENTION

In view of the above-mentioned shortcomings in the prior art, the object of the present application is to provide surgical auxiliary equipment for sutureless closed skin incisions in the superficial fascia of the skin, in order to solve the problems in the prior art that traces are easily left after the surgical incision is sutured and the effusions are difficult to discharge.

In one aspect, The surgical auxiliary equipment for sutureless closed skin incisions in the superficial fascia of the skin, comprising: a subcutaneous negative pressure drainage device, comprising a catheter partially reaching into a preset depth of an inner cavity of a subcutaneous incision, and used for generating a negative pressure, so that the inner cavity of the subcutaneous incision is in a closed state during the healing process; an incision closing device, arranged at a periphery of the skin incision, and used for squeezing the edge of the skin, so that the skin incision is maintained in a closed state during the healing process; and a negative pressure device above skin, arranged at the periphery of the skin incision, and used for generating a negative pressure to maintain the tissue position at the skin incision and the peripheral area to be stable.

In some embodiments, wherein the catheter partially reaches into the preset depth of the inner cavity of the subcutaneous incision via a preset position of the skin incision.

In some embodiments, wherein the catheter passes through the skin and subcutaneous tissue to partially reach into the preset depth of the inner cavity of the subcutaneous incision via a preset position far away from the skin incision.

In some embodiments, wherein the catheter is also used for draining effusions in the inner cavity of the subcutaneous incision through the generated negative pressure.

In some embodiments, wherein the subcutaneous negative pressure drainage device also comprises a negative pressure device used to generate and control the negative pressure, and the negative pressure device is communicated with the catheter.

In some embodiments, wherein the catheter is also used for delivering a liquid drug to the preset depth of the inner cavity of the subcutaneous incision.

In some embodiments, also comprising a drug delivery device used for delivering the liquid drug to the preset depth of the inner cavity of the subcutaneous incision through the catheter. In some embodiments, the drug delivering device is used for intermittent delivery of liquid drug through the catheter to a preset depth of the subcutaneous wound cavity.

In some embodiments, wherein the preset depth is an interval depth from the superficial fascia of the skin to a fat layer and a deep fascia layer in the skin tissue.

In some embodiments, wherein the preset depth is a junction between the fat layer and the deep fascia layer in the skin tissue.

In some embodiments, wherein the catheter comprises a first catheter and a second catheter separated from the first catheter, the first catheter is communicated with the negative pressure device, and the second catheter is communicated with the drug delivery device.

In some embodiments, wherein the first catheter and the second catheter are integrally formed.

In some embodiments, wherein the second catheter is sleeved in the first catheter.

In some embodiments, wherein the wall of the part of the catheter reaching into the preset depth of the skin incision is provided with a plurality of through holes.

In some embodiments, wherein the incision closing device comprises at least two closure members which are respectively arranged on the edges of both sides of the skin incision and used for squeezing the edge of the skin, so that the skin incision is maintained in a closed state during the healing process.

In some embodiments, wherein the incision closing device also comprises a micropore coverage member covering the closure member and the skin incision to adsorb effusions secreted from the skin incision.

In some embodiments, wherein the micropore coverage member is combined on the closure member according to the shape and structure of the closure member.

In some embodiments, wherein the micropore coverage member and the closure member are of an integrally formed structure.

In some embodiments, wherein the material of the micropore coverage member is foam, a mesh-shaped object, gauze, sponge or a porous biocompatible material.

In some embodiments, wherein the closure member comprises: a flexible body, adhered to the surface of the skin at one side of the skin incision; and a rigid curved needle, whose root is arranged on the flexible body and whose curved needle part is exposed out of the flexible body, and used for puncturing into one side of the skin incision to squeeze the edge of the skin so that the skin incision is maintained in a closed state during the healing process.

In some embodiments, wherein the closure member also comprises a clamping member used for clamping the flexible body adhered to both sides of the skin incision to provide an opposite force to squeeze the edge of the skin so that the skin incision is maintained in a closed state during the healing process.

In some embodiments, wherein the flexible body is made of a resin material or a silica gel material.

In some embodiments, wherein the closure member comprises: a first combining part formed on a flexible body; and a second combining part, correspondingly combined with the first combining part, and formed on another flexible body; wherein an opposite force applied to the flexible body at both sides of the skin incision is provided by virtue of combination of the first combining part and the second combining part to squeeze the edge of the skin, so that the skin incision is maintained in a closed state during the healing process.

In some embodiments, wherein the first combining part is of a groove structure or a buckle structure, and the second combining part is of a protruding structure that can be limited in the groove structure, or a hole or hook structure corresponding to the buckle structure.

In some embodiments, wherein the incision closing device also comprises an auxiliary member used for adhering to the at least two closure members and the surface of the skin and providing a contraction force for closing the skin incision to the at least two closure members. In some embodiments, wherein the auxiliary member comprises an adhesive tape.

In some embodiments, wherein the negative pressure device above skin comprises: a sealing membrane for adhering to the skin and covering the incision closing device to form a sealed space; and a negative pressure channel communicated with a negative pressure device, wherein the sealing membrane provides a contraction force through the negative pressure generated by the negative pressure device to drive the contraction of the skin incision, so as to help the incision closing device to close the skin incision.

In some embodiments, wherein the sealing membrane is adhered to the surface of the skin through an adhesive layer relative to the surface of the skin, and the material of the adhesive layer comprises a flexible impermeable material of polyurethane coated with an acrylic acid adhesive.

In some embodiments, wherein the sealing membrane is provided with an observation window made of a light transmitting material.

In some embodiments, wherein the negative pressure device above skin comprises a humidity detection component, used for providing detected humidity information in the sealed space to the negative pressure device, to facilitate the negative pressure device to regulate the negative pressure output.

In some embodiments, wherein the negative pressure device above skin is also used for allowing the sealing membrane to apply a force to a skin tissue by virtue of the negative pressure that generated, so as to assist in forcing the inner cavity of the subcutaneous incision to be in a closed state during the healing process.

In some embodiments, wherein the negative pressure device above skin and the subcutaneous negative pressure drainage device share a negative pressure device.

In some embodiments, wherein the catheter comprises subcutaneous drainage holes and negative pressure holes above skin, the subcutaneous drainage holes are a plurality of through holes formed on the wall of the part of the catheter reaching into the preset depth in the skin incision; and the negative pressure holes above skin are communicated with the sealed space to apply a negative pressure to the sealed space.

As described above, in the present application, in a first aspect, the surgical auxiliary equipment for sutureless closed skin incisions in the superficial fascia of the skin allows two ends of an exposed skin incision to be closely adhered through an incision closing device, the incision closing device can play a role of squeezing the edge of the skin while closing the incision, thereby reducing bleeding of vascular network under the skin dermis; in a second aspect, under the pressure of a subcutaneous negative pressure drainage device, the inner cavity of the subcutaneous incision is forced to be maintained in a closed state during the healing and rehabilitation process, the transecting tissues are kept in a fitting state, and the oozed blood and oozed fluid in the incision cavity are timely cleared through continuous negative pressure suction; on this basis, by intermittently delivering the liquid drug to the inner cavity of the subcutaneous incision, the solidified blood clots in the inner cavity of the subcutaneous incision can be moisturized to facilitate removal, and the potential bacterial community reaching a colonization concentration is diluted and removed along with the irrigation solution in drainage, so as to maintain the cleaning state of the incision cavity; in a third aspect, a negative pressure can be generated through the negative pressure device above skin to maintain the tissue position at the skin incision and its peripheral area to be stable, so as to facilitate healing of the tissue. In addition, through the surgical auxiliary equipment of the present application, sutureless closure of full-thickness tissues above the deep fascia of the skin can be achieved so as to avoid transverse scars (commonly known as "centipede foot") on the surface of the skin caused by suture oppression and cutting, and no suture knots are left in the superficial fascia so as to eliminate an important factor causing bacterial colonization and a main inducement leading to relapse of incision infection.

### BRIEF DESCRIPTION OF THE DRAWINGS

Fig. 1 is a schematic diagram of surgical auxiliary equipment for sutureless closed skin incisions in the superficial fascia of the skin of the present application in an embodiment.
Fig. 2 is a schematic diagram of a subcutaneous negative pressure drainage device of the present application in an embodiment.
Fig. 3 is a schematic diagram of a subcutaneous negative pressure drainage device of surgical auxiliary equipment of the present application in another embodiment.
Fig. 4 is a schematic diagram of a catheter in Fig. 3 passing through the skin and subcutaneous tissues from a preset position far away from the skin incision to partially reach into a long and narrow incision.
Fig. 5 is a structural schematic diagram of a closure member of an incision closing device of the present application in an embodiment.
Fig. 6 is a schematic diagram of combination of a closure member and an incision of an incision closing device of the present application in another embodiment.
Fig. 7 is a schematic diagram of a closure member of an incision closing device of the present application in still another embodiment.
Fig. 8 is an application schematic diagram of an auxiliary member of a closure member of an incision closing device of the present application in an embodiment.
Fig. 9 is a schematic diagram of an embodiment of a catheter structure of the present application.
Fig. 10 is a schematic diagram of another embodiment of a catheter structure of the present application.
Fig. 11 is a structural schematic diagram of a closure member of an incision closing device of the present application in yet another embodiment.
Fig. 12 is a schematic diagram of a sealing and stress structure of a negative pressure device above skin of the present application in an embodiment.
Fig. 13 is a schematic diagram of surgical auxiliary equipment for sutureless closed skin incisions in the superficial fascia of the skin of the present application in another embodiment.
Fig. 14 is a schematic diagram of surgical auxiliary equipment for sutureless closed skin incision in the superficial fascia of the skin of the present application in yet another embodiment.

### DETAILED DESCRIPTION OF THE EMBODIMENTS

Implementations of the present application will be described below through specific embodiments, and those skilled in the art can easily understand other advantages and effects of the present application from the contents disclosed in the present specification.

In the following description, several embodiments of the present application are described with reference to the attached figures. It should be understood that other embodiments may also be used, and changes of mechanical composition, structure, electric and operation may be made without departing from the spirit and scope of this application. The following detailed description should not be considered restrictive, and the scope of the implementation of this application is limited only by the published patent claims. The terminology used herein is intended only to describe specific embodiments and is not intended to restrict this application. Spatial terms, such as "up", "down", "left", "right", "lower part", "higher part", "bottom", "below", "above", "upwards", etc., can be used in this application to illustrate the relationship between one element and another or one feature and another shown in the figure.

Although in some embodiments the terms first and second are used in this article to describe various elements or parameters, these elements or parameters should not be restricted by these terms. These terms are used only to distinguish one element or parameter from another element or parameter. For example, the first catheter may be referred to as the second catheter, and similarly, the second catheter may be referred to as the first catheter without leaving the scope of the various described embodiments. Both the first catheter and the second catheter describe a catheter, but they are not the same catheter unless the context otherwise makes it clear.

Furthermore, as used herein, such single forms as "one", "a" and "the" aim at also including the plural forms, unless contrarily indicated in the text. It should be further understood that, such terms as "comprise" and "include" indicate the existence of the features, steps, operations, elements, components, items, types and/or groups, but do not exclude the existence, emergence or addition of one or more other features, steps, operations, elements, components, items, types and/or groups. The terms "or" and "and/or" used herein are explained to be inclusive, or indicate any one or any combination. Therefore, "A, B or C" or "A, B and/or C" indicates "any of the following: A; B; C; A and B; A and C; B and C; A, B and C". Exceptions of the definition only exist when the combinations of elements, functions, steps or operations are mutually exclusive inherently in some ways.

Surgeons used to divide the skin into a superficial fascia layer and a deep fascia layer. The superficial fascia refers to skin epidermis, dermis and subcutaneous fat tissues. Specifically, the superficial fascia is formed by continuation of the superficial fascia in the anterior and lateral regions of the chest and the superficial fascia of the neck, abdomen and upper limbs, and contains fat, superficial blood vessels, lymphatic vessels, cutaneous nerves and mammary glands. The deep fascia refers to fibrous connective tissues that separate the skin from muscle tissues. During the surgery, in the skin suturing stage, the superficial fascia and the deep fascia are usually sutured layer by layer in an order that the deep fascia is sutured first, then the subcutaneous tissue is sutured, and finally the skin is sutured. The significance of layered suturing is to resist against skin tension layer by layer in order to improve the quality of healing. Since skin suture can cause ischemia of local tissues, scars, commonly known as "centipede foot", may be formed in the direction perpendicular to the incision at the suture site after surgery, which is an inevitable result brought by the traditional suturing method. In addition, the conventional skin suturing also necessarily leave a large number of knots in the subcutaneous superficial fascia layer, which often leads to incision complications due to the existence of knots, such as incision infection, fat liquefaction, etc., thereby causing serious consequences and affecting the quality of life of sufferers.

As to the treatment of incisions (also known as incisions or surgical incisions, wounds, etc.) inevitably caused during surgeries of patients (sufferers), suturing is often used to help healing of the incisions of patients. In the treatment process, that is, after the incision is sutured with needles and threads, in order to facilitate healing of the incision, the decompression dressing components are applied in the closure force in the industry, the decompression dressing component comprises a pillow body formed by a closed pillow material and having a closing member, and the closing member can generate an inward closing force when the closed dressing pillow is placed under decompression. In some cases, the component also comprises a wicking material having a fluid flow path for removing fluid. However, this method still needs to suture the incision in the application process, which can only play a role in helping suturing and healing, and cannot completely replace the suturing step, so it will still leave suturing traces on the skin of the patient. In addition, the closing force produced in the structure can only act on the surface layer. When the incision is deep, the deep subcutaneous muscle tissue cannot be stressed to be in a closed state, and the wicking material can only deal with the fluid oozed from the surface of the skin, and cannot deal with subcutaneous effusions (for example, oozed blood and oozed fluid) in time, thereby being not conducive to the recovery of the incision.

In view of this, the present application provides surgical auxiliary equipment for sutureless closed skin incisions in the superficial fascia of the skin, in order to replace the traditional needle and thread suturing method for superficial fascia of the skin and achieve the purpose of closing the skin incision. In the following embodiments, the surgical auxiliary equipment for sutureless closed skin incisions in the superficial fascia of the skin of the present application comprises: a subcutaneous negative pressure drainage device, an incision closing device and a negative pressure device above skin, and is used for squeezing the edge of the skin through the incision closing device and allowing the inner cavity of the subcutaneous incision to be forced to be maintained in a closed state during the healing and rehabilitation process under the pressure of the subcutaneous negative pressure drainage device. The subcutaneous negative pressure drainage device can deliver a liquid drug to the inner cavity of the subcutaneous incision while draining the effusions in the inner cavity of the subcutaneous incision, so as to ensure that the environment in the inner cavity of the subcutaneous incision is maintained in a state beneficial to recovery of the incision. Meanwhile, the negative pressure device above skin can generate an acting force through the negative pressure device, so that the tissue position in the skin incision and its peripheral area are maintained in a stable state, and further the purpose of sutureless closure of skin incisions in the superficial fascia of the skin can be achieved.

In the present application, in a first aspect, the surgical auxiliary equipment for sutureless closed skin incisions in the superficial fascia of the skin allows two ends of an exposed skin incision to be closely adhered through an incision closing device, and the incision closing device can play a role of squeezing the edge of the skin while closing the incision, thereby reducing bleeding of the vascular network under skin dermis; in a second aspect, under the pressure of a subcutaneous negative pressure drainage device, the inner cavity of the subcutaneous incision is forced to be maintained in a closed state during the healing and rehabilitation process, the transecting tissues are kept in a fitting state, and the oozed blood and oozed fluid in the incision cavity are timely cleared through continuous negative pressure suction; on this basis, by intermittently delivering the liquid drug to the inner cavity of the subcutaneous incision, the solidified blood clots in the inner cavity of the subcutaneous incision can be moisturized to facilitate removal, and the potential bacterial community reaching a colonization concentration is diluted and removed along with irrigation solution in drainage, so as to maintain a cleaning state in the incision cavity; in a third aspect, a negative pressure can be generated through the negative pressure device above skin to maintain the tissue position at the skin incision and its peripheral areas to be stable, so as to facilitate healing of the tissue. In addition, through the surgical auxiliary equipment of the present application, sutureless closure of full-thickness tissues above the deep fascia of the skin can be achieved, so as to avoid transverse scars on the surface of the skin caused by suture oppression and cutting, and no suture knots are left in the superficial fascia, so as to eliminate an important factor causing bacterial colonization and a main inducement leading to relapse of incision infection.

It should be understood that, in embodiments concerned in the present application, the skin incision comprises cracks formed by continuous interruption of all the skins or other tissue parts, and widely refers to incisions, wounds, damages or other therapeutic targets located on or in tissues. It should be noted that in most cases, the skin incision is caused by surgery. However, in some cases, the skin incision may also be caused by accidents such as cuts or collisions.

In some embodiments, the tissues comprise but are not limited to bone tissues, fat tissues, muscle tissues, nerve tissues, skin tissues, vascular tissues, connective tissues, cartilages, tendons or ligaments. The incision can comprise, for example, chronic, acute, traumatic, subacute and dehiscent incisions; partial cortical burns and ulcers (such as diabetic ulcers, pressure ulcers or venous insufficiency ulcers), flaps, and grafts. The term "tissue part" can also refer to any tissue area that is not necessarily injured or damaged, but is an area in which it may wish to increase or promote the growth of other tissues. For example, a negative pressure can be applied to the tissue part so as to grow additional tissues that can be obtained and transplanted.

It should be understood that sutureless in the present application refers to a treatment means in which no needles and no suturing threads are used to suture the superficial fascia of the skin in the treatment of closing skin incision of the superficial fascia of the skin (i.e., skin epidermis, dermis and subcutaneous fat tissue parts) of the skin or the above superficial fascia layer of the skin, or in the healing process of the skin incision, or in other treatments of the skin incisions such as washing, disinfecting and topical application of drug after surgeries. In these processes, medical tools such as suturing needles and suturing threads are not used, therefore after the skin incision is healed, there is no process of clearing foreign matters in the incision or the incision surface of the superficial fascia of the skin, for example, a step and operating process of dismantling or removing the sutures or taking out the suture residues.

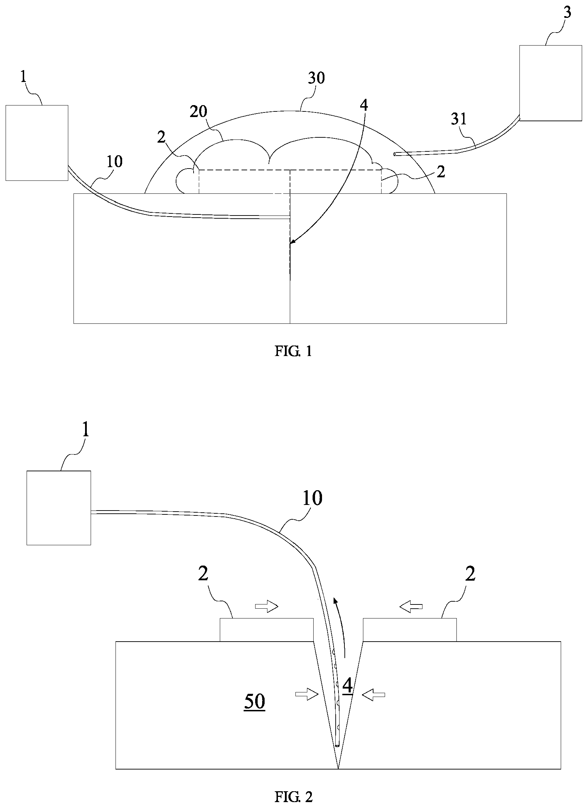

In one exemplary embodiment, please refer to Fig. 1, and Fig. 1 is a schematic diagram of surgical auxiliary equipment of the present application in an embodiment. As shown in the figure, the surgical auxiliary equipment for sutureless closed skin incisions in the superficial fascia of the skin of the present application comprises: a subcutaneous negative pressure drainage device 1, an incision closing device 2 and a negative pressure device above skin 3. In an exemplary embodiment, please refer to Fig. 2 which is a schematic diagram of a subcutaneous negative pressure drainage device of surgical auxiliary equipment of the present application in an embodiment. As shown in the figure, the subcutaneous negative pressure drainage device 1 comprises a catheter 10 partially reaching into a preset depth of the inner cavity 4 of the subcutaneous incision, and the catheter 10 is used for generating a negative pressure to force the inner cavity 4 of the subcutaneous incision to be in a closed state during the healing process, maintain the transecting tissue in a fitting state, and keep the oozed blood and oozed fluid in the incision cavity to be timely cleared through continuous negative pressure suction. In the actual implementation process, the catheter 10 of the subcutaneous negative pressure drainage device 1 reaches into the inner cavity 4 of the subcutaneous incision, the tissues at both sides of the inner cavity 4 of the subcutaneous incision are oppositely (as shown by the arrows on both sides of the subcutaneous incision 4 in Fig.2) fit by generating a negative pressure to eliminate the inner cavity 4 of the subcutaneous incision, and the closure of the inner cavity 4 of the subcutaneous incision facilitates healing of the incision. It should be understood that in this process, the part of the catheter 10 in the inner cavity 4 of the subcutaneous incision does not affect the growth of the tissue on both sides of the inner cavity 4 of the subcutaneous incision.

In another exemplary embodiment, please continue to refer to Fig. 2. As shown in the figure, the catheter 10 partially reaches into a preset depth of the inner cavity 4 of the subcutaneous incision through a preset position of the skin incision, so as to generate a negative pressure to ensure that the inner cavity 4 of the subcutaneous incision is in a closed state during the healing process, and meanwhile play a role in draining the effusions in the inner cavity 4 of the subcutaneous incision and further maintain the transecting tissue in a fitting state. The oozed blood and oozed fluid in the incision cavity are timely cleared through continuous negative pressure suction, so that since for example effusions such as oozed blood and/or oozed fluid of the inner cavity 4 of the subcutaneous incision can be timely cleared, the living space or environment of bacteria is eliminated. In the present embodiment, the preset position can be any position on the incision, and the preset position is arranged near the edge of the incision, which is conducive to recovery of the incision.

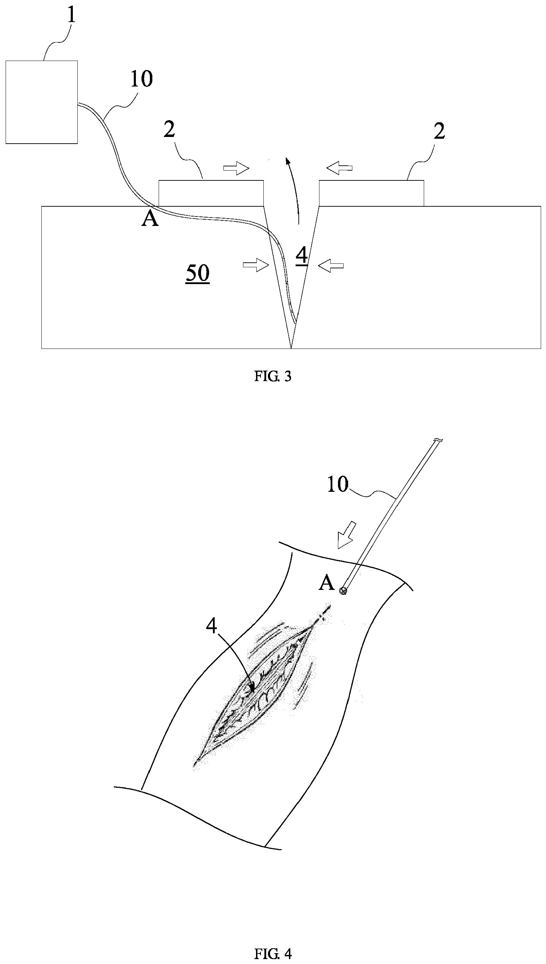

In another exemplary embodiment, please refer to Fig. 3 which is a schematic diagram of a subcutaneous negative pressure drainage device of surgical auxiliary equipment of the present application in another embodiment. As shown in the figure, the catheter 10 passes through the skin and the subcutaneous tissue via a preset position far away from the skin incision (a position as shown at A in Fig. 3) to partially reach into a preset depth of the inner cavity 4 of the subcutaneous incision, so as to generate a negative pressure to ensure that the effusions in the inner cavity 4 of the subcutaneous incision are sucked while the inner cavity 4 of the subcutaneous incision is in a closed state during the healing process, so that since the effusions in the inner cavity 4 of the subcutaneous incision can be timely cleared, the living space or environment of bacteria is eliminated. In the present embodiment, the distance refers to a preset position far away from the skin incision (a position as shown at A in Fig. 3). In order to facilitate closure treatment and surface treatment of the skin incision, the catheter 10 passes into the inner cavity 4 of the subcutaneous incision from another position rather than the skin incision, i.e., the catheter 10 passes through the skin and subcutaneous tissue from a preset position far away from the skin incision, to partially reach into the inner cavity 4 of the subcutaneous incision. The present embodiment is especially suitable for a long and narrow incision as shown in Fig. 4.

In the embodiment shown in Fig. 1, the surgical auxiliary equipment squeezes the edge of the skin through the incision closing device 2, so that the two exposed ends of the skin incision 4 tightly cling with each other. Under the force and assistance of the subcutaneous negative pressure drainage device 1, the inner cavity 4 of the subcutaneous incision is maintained in a closed state during the healing and rehabilitation process, the liquid drug is delivered to the inner cavity 4 of the subcutaneous incision while the effusions in the inner cavity 4 of the subcutaneous incision are sucked, and then the potential infection focus reaching a colonization concentration in the inner cavity 4 of the subcutaneous incision is diluted and washed; meanwhile, the negative pressure device above skin 3 can maintain the tissue position of the skin incision and its peripheral area to be stable through the generated negative pressure, and further facilitate recovery of the incision.

It should be understood that the inner cavity 4 of the subcutaneous incision refers to a cavity formed by the internal cut under the incision during the surgery. The cavity comprises gaps formed by continuous interruption of all the skins or other tissue parts. Due to the elastic or stretching performance of biological tissues of a human body or animal body, in the actual state, the inner cavity 4 of the subcutaneous incision, namely, the gap, is not necessarily intuitively present as a cavity state or a hollow state. Therefore, the shape and size of the gap are not limited in the embodiments provided in the present application.

It should be understood that the "catheter" disclosed in the present application refers to a fact that components can be in fluid linkage with each other so as to provide a path for fluid (i.e., liquid and/or gas) transfer among these components. For example, these components can be in fluid linkage through a fluid conducting device (such as a pipe). The "catheter" used herein widely comprises a pipe, a pipeline, a hose, a conduit or other structures having one or more pipe cavities adaptive to deliver fluid between two terminals. Typically, the pipe has some flexible, long and narrow cylindrical structures, but the geometrical shape and rigidity can be changed. In some embodiments, many components can be linked by virtue of physical access to form a single structure as a whole, or are made of the same material. In addition, some fluid conducting devices can be modulated to other components or combined with other components in other manners.

In some embodiments, a plurality of through holes are arranged on the wall of the part of the catheter 10 reaching into a preset depth of the skin incision, to facilitate the suction of gas and fluid in the inner cavity 4 of the subcutaneous incision. In an exemplary embodiment, the plurality of through holes are evenly distributed at intervals on the wall of the part of the catheter 10 reaching into a preset depth of the skin incision, in particular, for a long and narrow incision, the inner cavity of the subcutaneous incision formed under the skin of the long and narrow incision is usually a long cavity or a gap, in order to ensure that the effusions or residual drug liquid secreted at each place of the long cavity or the gap are sucked by the catheter 10; in another exemplary embodiment, adapting to different types of skin incisions or different purposes of surgeries, the multiple through holes on the catheter 10 can also be designed with unequal spacing (that is, the multiple through holes are unevenly distributed on the wall of the part of the catheter 10 reaching into a preset depth of the skin incision).

In another exemplary embodiment, the shape and structure of the part of the catheter 10 reaching into a preset depth of the skin incision can also be designed according to actual needs, for example, according to different depths expected to reach into the skin incision or different tissue structures, and different thicknesses or different flexibility performances of the catheter 10, such as different thicknesses of the same catheter at different parts, or different materials of the same catheter at different parts, or even different flexibility performances of the same catheter at different parts.

In some embodiments, the preset depth of the part of the catheter 10 reaching into the inner cavity 4 of the subcutaneous incision refers to an interval depth from the superficial fascia layer to the deep fascia layer in the skin tissue. Wherein in a preferred embodiment, the preset depth position is at the junction between the fat layer and the deep fascia layer in the skin tissue. It should be understood that the skin tissue successively comprises a vascular network, a superficial fascia layer, fat, a deep fascia layer, muscles and bones. Therefore, the preset depth in the present embodiment comprises the depth interval from the superficial fascia layer to the fat and the deep fascia layer.

In an exemplary embodiment, the subcutaneous negative pressure drainage device 1 also comprises a negative pressure device, the negative pressure device is used for generating and controlling the negative pressure, and the negative pressure device is communicated with the catheter 10, so that through the negative pressure device, the magnitude of the negative pressure in the catheter 10 is adjusted, so as to avoid such conditions that the incision injury is caused by excessive negative pressure or the negative pressure is insufficient to drain the effusions.

In the present embodiment, a check valve is arranged on the catheter 10 communicated with the negative pressure device, so as to prevent the gas or liquid sucked into the catheter 10 from flowing back into the inner cavity 4 of the subcutaneous incision, which is not conducive to healing of the skin incision. In the embodiment, the check valve is a rubber part such as a duckbill valve or a conical valve, but is not limited thereto. The valve assembly mechanically or electrically controlled is also applicable to the present application.

In the present embodiment, the negative pressure supply of the negative pressure device, such as a negative pressure source, can be an air reservoir under negative pressure, or can be a manually or electrically driven device that can reduce the pressure in the sealing volume, such as a vacuum pump, a suction pump, a wall suction port that can be used in many medical and health care facilities, or a micro pump, a syringe, a static negative pressure device or any suitable active or passive suction source. The negative pressure supply can be accommodated in other components or can be used in combination with them, and these other components are for example sensors, processing units, alarm indicators, memories, databases, software, display devices, or user interfaces that further facilitate treatment. For example, in some embodiments, the negative pressure source can be combined with other components to form a treatment unit. The negative pressure supply can also have one or more supply ports which are configured to facilitate linking and coupling of the negative pressure supply to one or more distribution components.

It should be understood that the "negative pressure" disclosed in the present application ordinarily refers to a pressure less than a local ambient pressure, such as an ambient pressure in a local environment outside the sealed treatment environment provided by application of drug. In many cases, the local ambient pressure can also be an atmospheric pressure of a place where the tissue part is located. Alternatively, the pressure can be less than a fluid static pressure associated with the tissues at the tissue part. Unless otherwise indicated, the value of the pressure stated herein is a gage pressure. Similarly, an increase of the mentioned negative pressure typically refers to a reduction in an absolute pressure, while the reduction in the negative pressure typically refers to an increase of the absolute pressure. Although the amount and property of the negative pressure applied to the tissue part can be varied with treatment requirements, however, the pressure is totally in low vacuum, ordinarily called crude vacuum, which is between -5mmHg (-667Pa) and -500 mmHg (-66.7kPa). The common therapeutic range is between -75mmHg (-9.9kPa) and -300mmHg (-39.9kPa).

Surgical site infection (SSI for short) is a clinical problem in the world. After the skin is cut/cracked, the deep tissue of the human body will contact with the outside, and may be possibly contaminated by pathogenic bacteria. When the bacterial content in the tissue exceeds 105/g, bacterial colonization will occur, and the bacteria cannot be controlled by the human immune system. With the exponential proliferation of bacteria, tissue necrosis and inflammatory reaction gradually appear in the infected area, and local symptoms of redness, swelling, heat and pain appear; due to an incubation period from bacterial colonization to the appearance of infection symptoms, SSI is usually difficult to be effectively identified and timely treated in the early stage. Typical incision infection usually occurs 7-10 days after the surgery, starting from local redness and swelling, tenderness, and followed by ulceration and pus. Because surgical sutures are easy to be colonized by bacteria, it is very important to remove all the suture knots of subcutaneous suturing as much as possible after SSI, which is also the key to preventing the relapse of SSI. This is because the source of infection of relapsed SSI often comes from residual suture knots.

Therefore, in an exemplary embodiment of the present application, the auxiliary surgical equipment for sutureless closed skin incisions in the superficial fascia of the skin in the present application also comprises a drug delivery device (not shown in the figure), and the drug delivery device is used for delivering the liquid drug to a preset depth of the inner cavity 4 of the subcutaneous incision, to facilitate healing of the inner cavity 4 of the subcutaneous incision. In specific implementation state, the drug delivery device for example comprises a drug delivery device or a drug delivery machine comprising a micro pump or a syringe. In one mode of the present embodiment, the liquid drug can be intermittently delivered to the inner cavity of the subcutaneous incision by controlling the drug delivery device, so that the solidified blood clots in the inner cavity 4 of the subcutaneous incision can be moisturized to facilitate removal, and the potential bacterial community reaching a colonization concentration can be diluted and removed along with irrigation solution in drainage, so as to maintain the cleaning state of the inner cavity 4 of the subcutaneous incision. In the present embodiment, according to different conditions, treatment schemes and constitutions of patients, the intermittence is, for example, a delivery frequency for delivery of drugs in hours or in days.

During the healing process, the drug delivery device can deliver liquid drugs to the inner cavity 4 of the subcutaneous incision through the communicated catheter, and is used for diluting bacterial infection that may be or has been generated in the inner cavity 4 of the subcutaneous incision, so as to dilute the potential infection focus reaching a colonization concentration; meanwhile, the liquid drug delivered by the drug delivery device can also achieve the purpose of irrigating the infected part in the inner cavity 4 of the subcutaneous incision. After the irrigation is completed, the liquid drug in the inner cavity 4 of the subcutaneous incision is sucked away through the drainage function of the catheter of the subcutaneous negative pressure drainage device 1, and then the bacteria are drained and cleared, and the inner cavity 4 of the subcutaneous incision is in a cleaning state.

In the actual clinical treatment, depending on the judged infection situation in the inner cavity of the subcutaneous incision of a patient, the dosage of the drug delivery device can be controlled, the time for diluting the bacterial infection part can be controlled, the irrigation frequency of liquid drugs can be controlled, and the working frequency of the subcutaneous negative pressure drainage device 1 can be controlled, etc. For example, in an exemplary embodiment, the time for irrigating the liquid drug in the inner cavity 4 of the subcutaneous incision by the drug delivery device is for example 2-3 days, and the drug delivery device can be controlled by virtue of an integrated machine (such as a drug delivery machine).

In the embodiment, the liquid drug comprises but is not limited to hydrogen peroxide and normal saline.

The catheter can be a catheter formed by integrating two catheters, or two catheters independent of each other, for example, in an exemplary embodiment, the catheter 10 comprises a first catheter 101 and a second catheter 102 isolated from the first catheter 101, and the first catheter 101 is communicated with the negative pressure device and used for sucking the effusions in the inner cavity 4 of the subcutaneous incision; and the second catheter 102 is communicated with a drug delivery device and used for delivering liquid drugs to a preset depth of the inner cavity 4 of the subcutaneous incision.

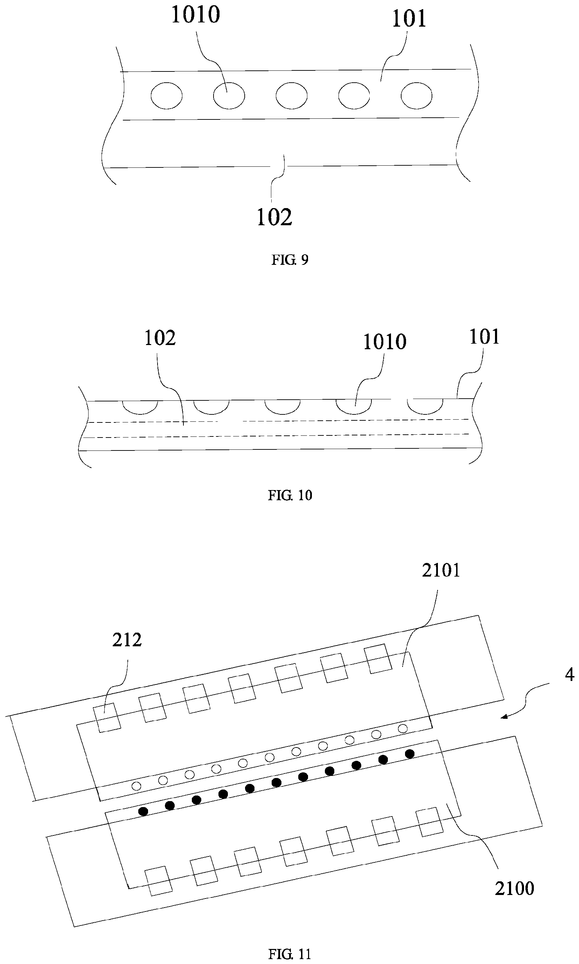

In an exemplary embodiment, please refer to Fig. 9 which is a schematic diagram of an embodiment of a catheter structure of the present application. As shown in the figure, the first catheter 101 and the second catheter 102 are integrally formed, so as to reduce the number of buried catheters. The integrally formed catheter comprises two mutually isolated and uncommunicated pipelines, wherein the first pipeline forms the first catheter 101, and the second pipe forms the second catheter 102, wherein the first catheter 101 is provided with a plurality of through holes 1010, the first catheter 101 is used for connecting the negative pressure device, and the second catheter 102 is used for connecting the drug delivery device. In an exemplary embodiment, please refer to Fig. 10 which is a schematic diagram of another embodiment of a catheter structure of the present application. As shown in the figure, the first catheter 101 and the second catheter 102 are integrally formed, so as to reduce the number of buried catheters. The integral formation means that the first catheter 101 and the second catheter 102 are two pipelines which are not communicated with each other, wherein the second catheter 102 is sleeved in the first catheter 101. Because the thinner catheter 102 is sleeved in the thicker catheter 101, the diameter of the whole catheter cannot be increased while the number of catheters is not increased. The thicker catheter 101 is provided with a plurality of through holes 1010, the thicker catheter 101 is used for connecting the negative pressure device, and the thinner catheter 102 is connected with the drug delivery device.

In some embodiments, the first catheter 101 is connected with the negative pressure device to generate negative pressure, the first catheter 101 arranged in the second catheter 102 is connected with the drug delivery device, the second catheter 102 is provided with a plurality of through holes 1010, to help to suck the gas and fluid in the inner cavity 4 of the subcutaneous incision.

In the embodiment provided by the present application, the incision closing device 2 is used for squeezing the edge of the skin so that the skin incision is in a closed state during the healing process. Meanwhile, squeezing the edge of the skin can reduce bleeding of the vascular network under the skin dermis, which is conducive to recovery of the incision. The incision closing device 2 is arranged at the periphery of the skin incision. In some embodiments, the incision closing device 2 is arranged at the periphery of the skin incision by means of adhesion. In some embodiments, the incision closing device 2 comprises at least two closure members which are respectively arranged on the edges of both sides of the skin incision. In the implementation process, the skin incision is maintained in a closed state during the healing process through the two closure members. In practical application, the number of closure members can be determined by the length of the incision and the specific form of closure members.

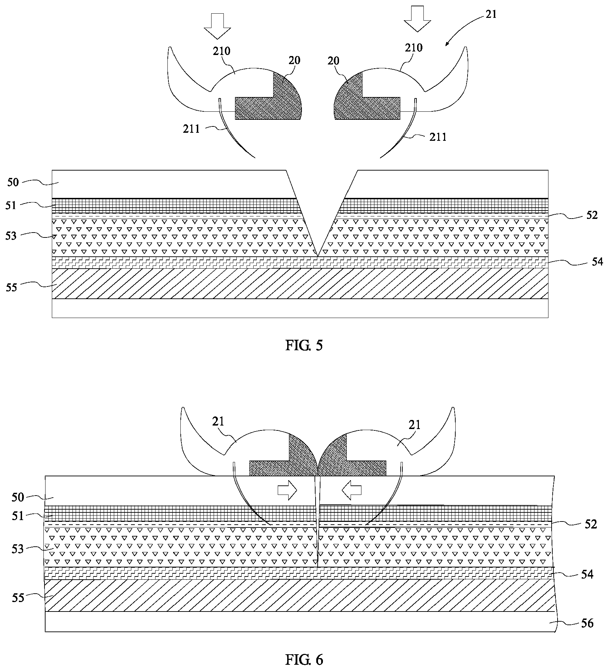

Please refer to Fig. 5 which is a structural schematic diagram of a closure member 21 of an incision closing device 2 of the present application in an embodiment. As shown in the figure, in the embodiment, the incision closing device 2 also comprises a microporous coverage member 20 covering the closure member 21, and the microporous coverage member 20 is combined on the closure member 21 according to the shape and structure of the closure member 21 and used to form an entirety with the closure member 21, so as to facilitate the adsorption or suction of the secretory fluid oozed from the skin incision and remaining on the closure member 21 or the drug liquid remaining on the closure member 21. In an exemplary embodiment, the microporous coverage member 20 and the closure member 21 are of an integrated structure. In another exemplary embodiment, for example, the microporous coverage member 20 is combined with the closure member 21 by bonding and other processes, so that the microporous coverage member 20 conforms to the shape and structure of the closure member 21 and is combined on the closure member 21.

In some embodiments, the closure member 21 comprises a flexible body which represents an elastic/flexible body material having more than 100% ultimate elongation and significant resilience magnitude. The resilience of the material is a capability of recovering the material from elastic deformation. The elastic/flexible body material for example may comprise but is not limited to natural rubber, polyisoprene, styrene butadiene rubber, chloroprene rubber, polybutadiene, nitrile butadiene rubber, butyl rubber, ethylene propylene rubber, ethylene propylene diene monomer, chlorosulfonated polyethylene, polysulfide rubber, polyurethane, EVA film, copolyester and silicone, etc.

In an exemplary embodiment, the closure member 21 may also be a silicon gel material, a resin material or a silica gel resin material.

In an exemplary embodiment, the material of the microporous coverage member 20 is medical cotton, degreasing cotton, foam, mesh, gauze, sponge, or porous biocompatible materials. The microporous coverage member 20 has air permeability and water adsorption feature, and is used for adsorbing the effusions possibly secreted from the skin incision during the healing process or water of the residual drug that is not absorbed by tissues and left on the skin incision.

In the present embodiment, the closure member 21 comprises a flexible body 210 and a rigid curved needle 211.

The flexible body 210 is adhered to the surface of the skin on the peripheral edge of the skin incision; in the present embodiment, the flexible body 210 is a silica gel material, a resin material or a silica gel resin material. The flexible body 210 is adhered to the surface of the skin on the peripheral edge of the skin incision through an adhesive. In some embodiments, the adhesive is for example a medical adhesive, for example comprising a rapid adhesive mainly based on methyl cyanoacrylate. In the specific implementation process, at least two flexible bodies 210 are respectively adhered to the surface of the skin at two relative sides of the peripheral edge of the skin incision.

The rigid curved needle 211 comprises a root and a curved needle part, wherein the root of the rigid curved needle 211 is buried in the flexible body 210 and firmly arranged on the flexible body 210, and the curved needle part of the rigid curved needle 211 is exposed out of the flexible body 210. In an exemplary embodiment, the root of the rigid curved needle 211 is firmly arranged in the flexible body 210 through a deformed structural design, such as a hook shaped structure or a T-shaped structure.

In an exemplary embodiment, please refer to Fig. 6 in combination with Fig. 5, which is shown as a schematic diagram of combination of a closure member and an incision of the present application in an embodiment. As shown in the figure, the curved needle part of the rigid curved needle 211 exposed out of the flexible body 210 punctures into one side of the skin incision to squeeze the edge of the skin, so that the skin incision is maintained in a closed state during the healing process. In the specific implementation process, doctors need to firstly align the incision (align the edge of the skin, in addition, prevent the edge of the skin from turning inwards to avoid poor skin healing), and then puncture the curved needle part of the rigid curved needle 211 from one side of the skin incision to be in a state as shown in Fig. 6. The rigid curved needle 211 of the closure member 21 on both sides of the skin incision punctures into the healthy tissue of the side edge of the incision, to squeeze the edge of the skin to reduce bleeding of the vascular network under the skin dermis, which is conducive to the recovery of the incision, so that the skin incision is in a closed state.

In another exemplary embodiment, the curved needle part of the rigid curved needle 211 exposed out of the flexible body 210 punctures into the other side of the skin incision, and the curved needle part is used to hook the other side of the skin incision, so both sides of the skin incision are oppositely combined, and meanwhile the healthy tissues on two opposite sides of the skin incision are driven to be oppositely combined to close the skin incision.

In another exemplary embodiment, the curved needle part of the rigid curved needle 211 exposed out of the flexible body 210 punctures into the flexible body 210 on the other side of the skin incision, so that the flexible bodies 210 on both sides of the skin incision are oppositely combined with each other, and meanwhile the healthy tissues on two opposite sides of the skin incision are driven to be oppositely combined to close the skin incision.

As shown in Fig. 6, a vascular network 51, a superficial fascia layer 52, fat 53, a deep fascia layer 54, muscles 55 and bones 56 are comprised in sequence under the tissue of the skin 50. The suturing depth in the present embodiment is in the superficial fascia layer 52. The problem concerned in the present application is sutureless operation of the superficial fascia part of the skin (i.e., the skin epidermis, dermis and subcutaneous fat tissue). The puncturing depth of the curved needle part exposed out of the flexible body 210 of the rigid curved needle 211 comprises the skin epidermis and dermis of the superficial fascia of the skin and subcutaneous fat tissues.

In some embodiments, depending on different lengths or widths of the applicable skin incisions, the number and length of the rigid curved needles 211 arranged on the flexible body 210 can present different implementation states. In some embodiments, when multiple rigid curved needles 211 are arranged on the flexible body 210, the rigid curved needles 211 are equidistantly arranged on the long-strip flexible body 210.

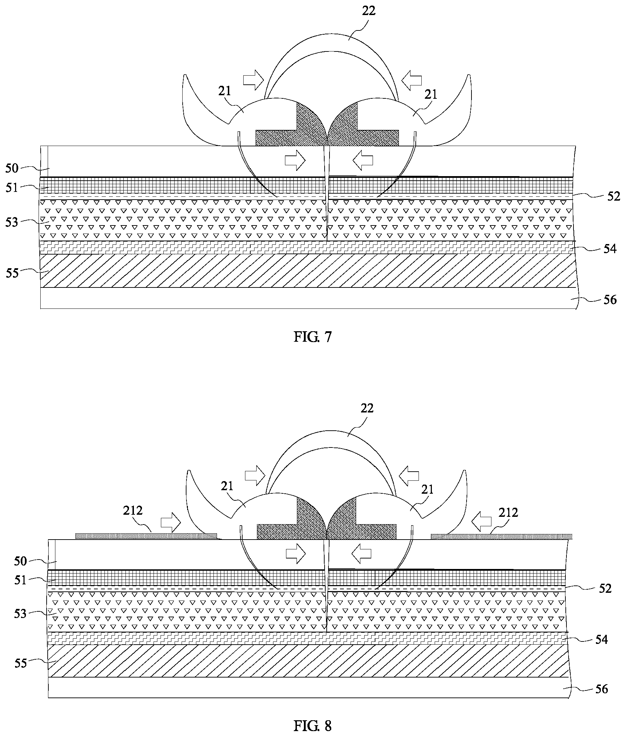

Please refer to Fig. 7 which is a schematic diagram of a closure member of an incision closing device of the present application in another embodiment. As shown in the figure, in the present embodiment, the closure member also comprises a clamping member 22, and the clamping member 22 is used for clamping the flexible body of the closure member 21 adhered to both sides of the skin incision. The flexible body provides an opposite force to squeeze the edge of the skin under the action of the clamping member, to ensure that the skin incision is in a closed state during the healing process. During specific implementation, the clamping member 22 is a medical clip or other parts.

In the above embodiments using the closure member 21 comprising the flexible body 210 and the rigid curved needle 211, some auxiliary members can also be used. The auxiliary members are used for assisting in adhesion between the closure member 21 and the surface of the skin, and providing a contraction force closing the skin incision to the closure member 21. Please refer to Fig. 8 which is an application schematic diagram of an auxiliary member of a closure member of an incision closing device of the present application in an embodiment.

In another exemplary embodiment, please refer to Fig. 11 which is a structural schematic diagram of a closure member of an incision closing device of the present application in yet another embodiment. As shown in the figure, in the present embodiment, the closure member 21 comprises a first flexible body 2100 and a second flexible body 2101.

The first flexible body 2100 is adhered to the surface of the skin at the periphery of the skin incision, and the first flexible body 2100 has a first combining part; the second flexible body 2101 is adhered to the surface of the skin at the periphery of the skin incision, and the second flexible body 2101 has a second combing part corresponding to the first combining part. The combination of the first combining part and the second combining part provides an opposite force applied to the flexible bodies on both sides of the skin incision, thereby squeezing the edge of the skin so that the skin incision is maintained in a closed state during the healing process. In the present embodiment, the first and second flexible bodies 2101 are made of a silica gel material, a resin material or a silica gel resin material. The first and second flexible bodies 2101 are adhered to the surface of the skins on two opposite sides of the skin incision by an adhesive. In some embodiments, the adhesive is a medical adhesive, such as a rapid adhesive based on methyl cyanoacrylate.

In some exemplary embodiments, the first combing part is of a groove structure or a buckle structure, and the second combing part is of a protruding structure that can be limited in the groove structure or a hole or hook structure corresponding to the buckle. For example, the first combining part is provided with a plurality of grooves, and the second combining part is provided with protruding structures corresponding to the grooves. A plurality of grooves on the first combining part can be replaced by a buckle structure, and correspondingly, the protruding structure on the second combining part can be replaced by holes or a hook structure corresponding to the buckle; but it is not limited thereto. Other combining structures that can realize the combination of the first flexible body 2100 and the second flexible body 2101 can achieve the purpose of the present embodiment, such as the combination of a groove or a hole and a protruding structure, etc.

In some embodiments, please continue to refer to Fig. 11. The incision closing device 2 also comprises an auxiliary member 212, and the auxiliary member 212 is used for assisting in adhesion between the closure member 21 and the surface of the skin, and providing a contraction force closing the skin incision to the closure member 21. In an exemplary embodiment, if the skin incision is an incision in the first direction, the auxiliary member 212 is adhered to the periphery of the skin incision and applies a force in the second direction vertical to the first direction to the closure member 21, and this force is called a contraction force in the present embodiment, to help the closure member 21 to close the skin incision. In the present embodiment, the auxiliary member 212 is adhered to the surface of the skin through an adhesive layer relative to the surface of the skin, and the material of the adhesive layer for example comprises an adhesive tape of polyurethane coated with an acrylic bonding agent.

In the present embodiment, the auxiliary member 212 of the adhesive tape can be designed as a belt structure extending from the center to the two opposite sides, the belt structure extending from both sides can be separated or integrated, and the belt structure is suitable for artificial extension in order to increase the comfort level of patients/sufferers.

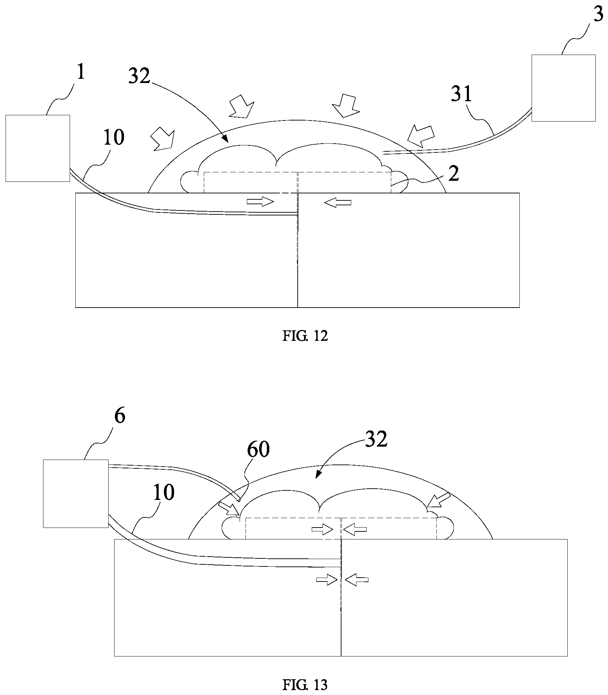

In some embodiments, the negative pressure device above skin 3 comprises a sealing membrane and a negative pressure channel. Please refer to Fig. 12 which is a sealing and stress structural schematic diagram of a negative pressure device above skin of the present application in an embodiment. As shown in the figure, the sealing membrane 30 is adhered to the skin and covers the incision closing device 2, thereby forming a sealed space 32. After being pumped by the negative pressure device, an inward acting force is formed in the sealed space inside the sealing membrane, and the stress is in the direction of the arrow shown in Fig. 12. A pressure can be applied to the deep tissues while maintaining local tissue position stationary, so as to close the potential dead cavity. In the present embodiment, the sealing membrane 30 forms a sealing area around the skin incision and the incision closing device 2 arranged on the skin incision, and makes the sealing area form a sealed space 32. In the present embodiment, the sealing membrane 30 is adhered to the surface of the skin through the adhesive layer relative to the surface of the skin. The material of the adhesive layer is for example a flexible impermeable material comprising polyurethane coated with an acrylic bonding agent.

In an exemplary embodiment, the sealing membrane 30 can be made of a transparent material. The sealing membrane 30 made of the transparent material can facilitate clinicians to visually observe the healing of the skin incision, so as to perform timely intervention.

In an exemplary embodiment, the sealing membrane 30 is provided with an observation window made of a light transmitting material, to facilitate clinicians to observe the healing of the skin incision and take corresponding medical measures.

For specific operation steps of the above embodiment, please refer to Fig. 1. As shown in the figure, the operator embeds the catheter 10 of the subcutaneous negative pressure drainage device into the subcutaneous tissue of the skin, and extends the catheter 10 into the inner cavity 4 of the subcutaneous incision. The subcutaneous negative pressure drainage device can drain the effusions secreted from the inner cavity 4 of the subcutaneous incision through the catheter 10, and meanwhile the action of the negative pressure forces the inner cavity of the incision to be closed/combined, so that tissues at both sides are oppositely combined. In addition, the liquid drug can be delivered to the inner cavity 4 of the subcutaneous incision through the catheter, which is conducive to healing, the above is the first aspect; in a second aspect, the skin on both sides outside the incision are closed inwards by the closure member, so that the skin incision is maintained in a closed state during the healing process. The squeezing effect of the closure member on the edge of the skin can reduce bleeding of the vascular network under the skin dermis, and the negative pressure of the subcutaneous negative pressure drainage device can assist in the closure of the internal cavity 4 of the subcutaneous incision. The operator continues to cover the surface of the incision with the microporous coverage member 20, to ensure that the secreta on the surface of the incision can be absorbed in time during the healing process. Then, a sealing membrane 30 is covered outside the microporous accessory to wrap the whole incision and the microporous coverage member 20 on the incision; in a third aspect, the sealing membrane 30 is pumped through the negative pressure channel 31 of the negative pressure device above skin 3, so that tissues at the skin incision and its peripheral area are tightened towards the incision. In addition, the negative pressure generated by the negative pressure device above skin at the same time can also exert a certain degree of pressure on the deep tissue, so as to close the potential dead space, thereby speeding up the healing speed of the skin incision. A treatment means in which the superficial fascia of the skin is sutured without needles and threads is not used, and medical tools such as suturing needles and sutures are not used.

In the present embodiment, the negative pressure channel 31 is communicated with a negative pressure device through the catheter, and the negative pressure channel 31 is communicated with the sealed space, thereby providing a negative pressure source and forming a sealed space.

In the present embodiment, the negative pressure generated by the negative pressure device compresses the sealed space, so that the local tissue is maintained to be closed towards the incision position. At the same time, the negative pressure generated by the negative pressure device also exerts an acting force on the deep tissue under the incision, so that the potential dead space is closed, to help the incision closing device 2 to close the skin incision, and further promote the healing of the incision. In the present embodiment, the range of the pressure value of the sealed space formed by the sealing membrane 30 adhering to the skin can be set between about 0.001 atmospheric pressure and about 1 atmospheric pressure. In the actual implementation process, the negative pressure value generated by the negative pressure device can be controlled according to the healing degree of the incision, for example, the negative pressure value can be appropriately reduced along with the healing degree of the skin incision, or according to the situation of the effusions secreted by the skin incision, for example, the negative pressure value can be appropriately increased to enlarge the strength of sucking the effusions under the condition of increased secreted effusions.

In the present embodiment, the catheter by which the negative pressure channel 31 is communicated with a negative pressure device is provided with a check valve, to avoid that the gas or liquid sucked into the catheter flows back into the sealed space, which is not beneficial for the healing of the skin incision. In the embodiment, the check valve is a rubber part such as a duckbill valve or a cone-shaped valve, but is not limited thereto. Valve assemblies mechanically or electrically controlled are also applicable to the present embodiment.

In the present embodiment, the negative pressure supply of the negative pressure device, such as the negative pressure source, can be an air reservoir under the negative pressure, or can be a manually or electrically driven device that can reduce the pressure in the sealing volume, such as a vacuum pump, a suction pump, a wall suction port that can be used in many medical and health care facilities, or a micro pump, a syringe, a static negative pressure device or any suitable active or passive suction source. The negative pressure supply can be accommodated in other components or used in combination with these other components. These other components are for example sensors, processing units, alarm indicators, memories, databases, software, display devices, or user interfaces that further facilitate treatment. For example, in some embodiments, the negative pressure source can be combined with other components to form a treatment unit. The negative pressure supply can also have one or more supply ports which are configured to facilitate connection and coupling of the negative pressure supply to one or more distribution components.

In an exemplary embodiment, please refer to Fig. 13 which is a schematic diagram of surgical auxiliary equipment for sutureless closed skin incisions in the superficial fascia of the skin of the present application in another embodiment. As shown in Fig. 13, the negative pressure device above skin can share a negative pressure source 6 with the subcutaneous negative pressure drainage device. The negative pressure device above skin can be respectively connected to the same negative pressure source through two different catheters, i.e., a catheter communicated with the skin sealed space 32 and a catheter communicated with the inner cavity of the subcutaneous incision are two different catheters.

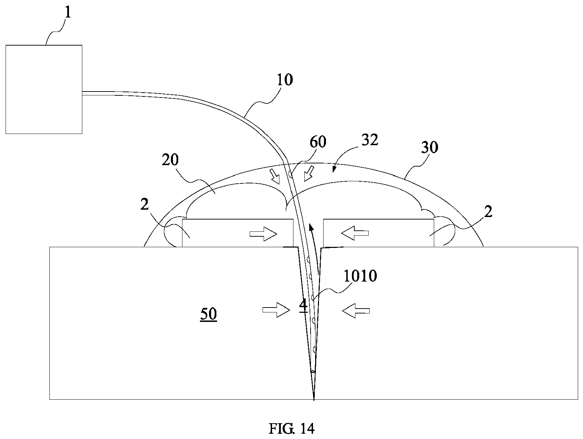

Please refer to Fig. 14 which is a schematic diagram of surgical auxiliary equipment for sutureless closed skin incision in the superficial fascia of the skin of the present application in yet another embodiment. As shown in the figure, in the present embodiment, the negative pressure device above skin and the subcutaneous negative pressure drainage device can share one negative pressure source 6 which is respectively communicated with the inner cavity 4 of the subcutaneous incision and the skin sealed space 32 through a catheter. Wherein the catheter 10 comprises subcutaneous drainage holes 1010 and negative pressure holes above skin 60. The subcutaneous drainage holes are a plurality of through holes 1010 formed on the wall of the part of the catheter reaching into a preset depth of the skin incision; and the negative pressure holes above skin are communicated with the sealed space to apply a negative pressure to the sealed space.

In an exemplary embodiment, the negative pressure device above skin 3 also comprises a humidity detection component (not shown in the figure), the sensor of the humidity detection component is arranged in the sealed space formed by the sealing membrane 30, and the information output port of the humidity detection component is connected with the negative pressure device, so as to provide the detected humidity information to the negative pressure device, to control the negative pressure device to regulate the magnitude of the output negative pressure. In the present embodiment, the humidity detection component is for example a humidity sensor.

To sum up, in a first aspect, the surgical auxiliary equipment for sutureless closed skin incisions in the superficial fascia of the skin in the present application allows two exposed ends of a skin incision to be closely adhered through an incision closing device, the incision closing device can play a role of squeezing the edge of the skin while closing the incision, thereby reducing bleeding of the vascular network under the skin dermis; on a second aspect, under the pressure of a subcutaneous negative pressure drainage device, the inner cavity of the subcutaneous incision is forced to be maintained in a closed state during the healing and rehabilitation process, the transecting tissues are kept in a fitting state, and the oozed blood and oozed fluid in the incision cavity are timely cleared through continuous negative pressure suction; on this basis, by intermittently delivering the liquid drug to the inner cavity of the subcutaneous incision, the solidified blood clots in the inner cavity of the subcutaneous incision can be moisturized to facilitate removal, and the potential bacterial community reaching a colonization concentration is diluted and removed along with the irrigation solution in drainage, so as to maintain the cleaning state of the incision cavity; in a third aspect, a negative pressure can be generated through the negative pressure device above skin to maintain the tissue position at the skin incision and its peripheral area to be stable, so as to facilitate healing of the tissue. In addition, through the surgical auxiliary equipment of the present application, sutureless closure of full-thickness tissues above the deep fascia of the skin can be achieved, so as to avoid transverse scars (commonly known as "centipede foot") on the surface of the skin caused by suture oppression and cutting, and no suture knots are left in the superficial fascia, so as to eliminate an important factor causing bacterial colonization and a main inducement leading to relapse of incision infection.

Therefore, the surgical auxiliary equipment for sutureless closed skin incisions in the superficial fascia of the skin can always maintain the inner cavity 4 of the subcutaneous incision in a closed state without sutures during the healing and rehabilitation process. Accordingly, not only the healing speed of the skin incision is quickened, but also a treatment means in which the skin is sutured without needles and threads is used, medical tools such as suturing needles and suture are not used, therefore, after the skin incision is healed, there is no process of clearing foreign matters in the incision or incision surface, for example, a step and operating process of dismantling or removing the sutures or taking out the thread residues, and then key links and important inducements for bacterial colonization are eliminated, and more importantly, it is ensured that the healed incision does not leave any traces on the surface of the skin, such as "centipede foot", and the surgical incision part is aesthetically pleasing, and further problems in the prior art that traces are easily left after the surgical incision is sutured and the effusions are difficult to discharge are solved. The surgical auxiliary equipment for sutureless closed skin incisions in the superficial fascia of the skin is especially applicable to the field of hairdressing.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

## Claims

1. Surgical auxiliary equipment for sutureless closed skin incisions in the superficial fascia of the skin, comprising:
a subcutaneous negative pressure drainage device, comprising a catheter partially reaching into a preset depth of an inner cavity of a subcutaneous incision, and used for generating a negative pressure, so that the inner cavity of the subcutaneous incision is in a closed state during the healing process;
an incision closing device, arranged at a periphery of the skin incision, and used for squeezing the edge of the skin, so that the skin incision is maintained in a closed state during the healing process; and
a negative pressure device above skin, arranged at the periphery of the skin incision, and used for generating a negative pressure to maintain the tissue position at the skin incision and the peripheral area to be stable.

2. The surgical auxiliary equipment for sutureless closed skin incisions in the superficial fascia of the skin according to claim 1, wherein the catheter partially reaches into the preset depth of the inner cavity of the subcutaneous incision via a preset position of the skin incision.

3. The surgical auxiliary equipment for sutureless closed skin incisions in the superficial fascia of the skin according to claim 1, wherein the catheter passes through the skin and subcutaneous tissue to partially reach into the preset depth of the inner cavity of the subcutaneous incision via a preset position far away from the skin incision.

4. The surgical auxiliary equipment for sutureless closed skin incisions in the superficial fascia of the skin according to claim 1, wherein the catheter is also used for draining effusions in the inner cavity of the subcutaneous incision through the generated negative pressure.

5. The surgical auxiliary equipment for sutureless closed skin incisions in the superficial fascia of the skin according to claim 1, wherein the subcutaneous negative pressure drainage device also comprises a negative pressure device used to generate and control the negative pressure, and the negative pressure device is communicated with the catheter.

6. The surgical auxiliary equipment for sutureless closed skin incisions in the superficial fascia of the skin according to claim 1, wherein the catheter is also used for delivering a liquid drug to the preset depth of the inner cavity of the subcutaneous incision.

7. The surgical auxiliary equipment for sutureless closed skin incisions in the superficial fascia of the skin according to claim 6, also comprising a drug delivery device used for delivering the liquid drug to the preset depth of the inner cavity of the subcutaneous incision through the catheter.

8. The surgical auxiliary equipment for sutureless closed skin incisions in the superficial fascia of the skin according to claim 1, 2, 3, 6 or 7, wherein the preset depth is an interval depth from the superficial fascia of the skin to a fat layer and a deep fascia layer in the skin tissue.

9. The surgical auxiliary equipment for sutureless closed skin incisions in the superficial fascia of the skin according to claim 8, wherein the preset depth is a junction between the fat layer and the deep fascia layer in the skin tissue.

10. The surgical auxiliary equipment for sutureless closed skin incisions in the superficial fascia of the skin according to claim 7, wherein the catheter comprises a first catheter and a second catheter separated from the first catheter, the first catheter is communicated with the negative pressure device, and the second catheter is communicated with the drug delivery device.

11. The surgical auxiliary equipment for sutureless closed skin incisions in the superficial fascia of the skin according to claim 10, wherein the first catheter and the second catheter are integrally formed.

12. The surgical auxiliary equipment for sutureless closed skin incisions in the superficial fascia of the skin according to claim 10, wherein the second catheter is sleeved in the first catheter.

13. The surgical auxiliary equipment for sutureless closed skin incisions in the superficial fascia of the skin according to claim 1, wherein the wall of the part of the catheter reaching into the preset depth of the skin incision is provided with a plurality of through holes.

14. The surgical auxiliary equipment for sutureless closed skin incisions in superficial fascia of the skin according to claim 1, wherein the incision closing device comprises at least two closure members which are respectively arranged on the edges of both sides of the skin incision and used for squeezing the edge of the skin, so that the skin incision is maintained in a closed state during the healing process.

15. The surgical auxiliary equipment for sutureless closed skin incisions in the superficial fascia of the skin according to claim 14, wherein the incision closing device also comprises a micropore coverage member covering the closure member and the skin incision to adsorb effusions secreted from the skin incision.

16. The surgical auxiliary equipment for sutureless closed skin incisions in the superficial fascia of the skin according to claim 15, wherein the micropore coverage member is combined on the closure member according to the shape and structure of the closure member.

17. The surgical auxiliary equipment for sutureless closed skin incisions in the superficial fascia of the skin according to claim 15, wherein the micropore coverage member and the closure member are of an integrally formed structure.

18. The surgical auxiliary equipment for sutureless closed skin incisions in the superficial fascia of the skin according to claim 15, wherein the material of the micropore coverage member is foam, a mesh-shaped object, gauze, sponge or a porous biocompatible material.

19. The surgical auxiliary equipment for sutureless closed skin incisions in the superficial fascia of the skin according to claim 14, wherein the closure member comprises:
a flexible body, adhered to the surface of the skin at one side of the skin incision; and
a rigid curved needle, whose root is arranged on the flexible body and whose curved needle part is exposed out of the flexible body, and used for puncturing into one side of the skin incision to squeeze the edge of the skin so that the skin incision is maintained in a closed state during the healing process.

20. The surgical auxiliary equipment for sutureless closed skin incisions in the superficial fascia of the skin according to claim 19, wherein the closure member also comprises a clamping member used for clamping the flexible body adhered to both sides of the skin incision to provide an opposite force to squeeze the edge of the skin so that the skin incision is maintained in a closed state during the healing process.

21. The surgical auxiliary equipment for sutureless closed skin incisions in the superficial fascia of the skin according to claim 19, wherein the flexible body is made of a resin material or a silica gel material.

22. The surgical auxiliary equipment for sutureless closed skin incisions in the superficial fascia of the skin according to claim 14, wherein the closure member comprises:
a first combining part formed on a flexible body; and
a second combining part, correspondingly combined with the first combining part, and formed on another flexible body; wherein
an opposite force applied to the flexible body at both sides of the skin incision is provided by virtue of combination of the first combining part and the second combining part to squeeze the edge of the skin, so that the skin incision is maintained in a closed state during the healing process.

23. The surgical auxiliary equipment for sutureless closed skin incisions in the superficial fascia of the skin according to claim 22, wherein the first combining part is of a groove structure or a buckle structure, and the second combining part is of a protruding structure that can be limited in the groove structure, or a hole or hook structure corresponding to the buckle structure.

24. The surgical auxiliary equipment for sutureless closed skin incisions in the superficial fascia of the skin according to claim 14, wherein the incision closing device also comprises an auxiliary member used for adhering to the at least two closure members and the surface of the skin and providing a contraction force for closing the skin incision to the at least two closure members.

25. The surgical auxiliary equipment for sutureless closed skin incisions in the superficial fascia of the skin according to claim 24, wherein the auxiliary member comprises an adhesive tape.

26. The surgical auxiliary equipment for sutureless closed skin incisions in the superficial fascia of the skin according to claim 1, wherein the negative pressure device above skin comprises:
a sealing membrane for adhering to the skin and covering the incision closing device to form a sealed space; and
a negative pressure channel communicated with a negative pressure device, wherein the sealing membrane provides a contraction force through the negative pressure generated by the negative pressure device to drive the contraction of the skin incision, so as to help the incision closing device to close the skin incision.

27. The surgical auxiliary equipment for sutureless closed skin incisions in the superficial fascia of the skin according to claim 26, wherein the sealing membrane is adhered to the surface of the skin through an adhesive layer relative to the surface of the skin, and the material of the adhesive layer comprises a flexible impermeable material of polyurethane coated with an acrylic acid adhesive.

28. The surgical auxiliary equipment for sutureless closed skin incisions in the superficial fascia of the skin according to claim 26, wherein the sealing membrane is provided with an observation window made of a light transmitting material.

29. The surgical auxiliary equipment for sutureless closed skin incisions in the superficial fascia of the skin according to claim 26, wherein the negative pressure device above skin comprises a humidity detection component, used for providing detected humidity information in the sealed space to the negative pressure device, to facilitate the negative pressure device to regulate the negative pressure output.

30. The surgical auxiliary equipment for sutureless closed skin incisions in the superficial fascia of the skin according to claim 26, wherein the negative pressure device above skin is also used for allowing the sealing membrane to apply a force to a skin tissue by virtue of the negative pressure that generated, so as to assist in forcing the inner cavity of the subcutaneous incision to be in a closed state during the healing process.

31. The surgical auxiliary equipment for sutureless closed skin incisions in the superficial fascia of the skin according to claim 26, wherein the negative pressure device above skin and the subcutaneous negative pressure drainage device share a negative pressure device.

32. The surgical auxiliary equipment for sutureless closed skin incisions in the superficial fascia of the skin according to claim 31, wherein the catheter comprises subcutaneous drainage holes and negative pressure holes above skin, the subcutaneous drainage holes are a plurality of through holes formed on the wall of the part of the catheter reaching into the preset depth in the skin incision; and the negative pressure holes above skin are communicated with the sealed space to apply a negative pressure to the sealed space.
